# EUROPEAN PATENT SPECIFICATION

(11) **EP 1 194 781 B1**
(45) Date of publication and mention of the grant of the patent: **07.03.2007**
(21) Application number: 00938417.3
(22) Date of filing: 15.06.2000
(51) Int. Cl.: G01N 33/533, G01N 33/58

(54) **METHOD FOR CONDUCTING CHEMILUMINESCENT BINDING ASSAY**
VERFAHREN ZUR PROZESSFÜHRUNG CHEMILUMINESZENTER BINDUNGSTESTS
PROCEDE DE DOSAGE PAR LIAISON CHIMILUMINESCENTE

(30) Priority: 18.06.1999 US 139941 P
(43) Date of publication of application: 10.04.2002
(73) Proprietor: Cardiogenics Inc., Toronto, Ontario M8Z 1J7 (CA)
(72) Inventor: GAWAD, Yahia, Mississauga Ontario L4W 2X3 (CA)
(74) Representative: Mallalieu, Catherine Louise
(86) International application number: PCT/CA2000/000718
(87) International publication number: WO 2000/079276

(56) References cited:
- EP-A- 0 437 013
- WO-A-98/30908
- WO-A-99/38999
- US-A- 5 486 455
- KENDALL J M ET AL: "Aequorea victoria bioluminescence moves into an exciting new era" TRENDS IN BIOTECHNOLOGY,GB,ELSEVIER PUBLICATIONS, CAMBRIDGE, vol. 16, no. 5, 1 May 1998 (1998-05-01), pages 216-224, XP004117786 ISSN: 0167-7799

## Description

### Field of the Invention

The present invention relates to a method for conducting a binding assay, and in particular to an immunoassay method that may be conducted on a Point Of Care (POC) device or an autoanalyzer.

### Background to the Invention

The on-going needs to detect and quantify biomolecules (analytes) in various body fluids have resulted in the introduction of new and more accurate analytical techniques that can be adapted for measuring a wide spectrum of different analytes. Most of these detection methods have been introduced into the clinical diagnostic field in recent years. Currently, a broad expansion in both the variety of analytes that may be readily and accurately determined as well as the methods for the determination have been witnessed. However, convenient, reliable, non-hazardous, highly sensitive and technically less challenging methods for detecting the presence of low concentrations of analytes in liquids are still desired, especially when the analyte may be present in body fluids in very low concentrations.

Several methods for the detection and quantification of substances of biological origin in fluid samples are currently employed. Bioanalytical assays, such as immunoassays and nucleic acid hybridization assays, which are based on the specific binding between ligands and one or more members of specific binding pairs are widely used to determine the presence and quantity of analytes of interest, for example chemical constituents or substances of a sample. In particular, immunoassays are widely employed detection and quantification methods in the clinical laboratory.

In a typical procedure of a sandwich immunoassay, an antibody against a particular antigen, known as a capture antibody, is immobilized to a solid surface. The sample under investigation is contacted with the solid surface under conditions that allow antigen in the sample to bind to the capture antibody. Another antibody known as a detector antibody is added. In the direct immunoassay format, the detector antibody is directly conjugated with a signal generating mechanism that allows the amount of the detector antibody to be quantified. In the indirect format, after the binding of the detector antibody to the antigen, another antibody against the detector antibody or another specific binding reaction that involves the detector antibody is utilized. This so-called anti-detector antibody is directly conjugated with a signal generating mechanism. The binding reaction and therefore the antigen level in the sample is quantified by quantifying the signal produced by the signal generating mechanism.

Several types of labeling material have been utilized for signal generation in the receptor-ligand binding assays. Radioactive atoms, such as ¹²⁵I, ¹³¹I, ³H and ¹⁴C were commonly utilized as the label. Although radioactive labels for immunoassays are sensitive, they suffer commonly recognized disadvantages, including safety and the stringent regulatory requirements resulting in a relatively short reagent shelf life. Several alternative labeling methods are currently utilized in binding bioassays including colorimetric enzyme reactions, fluorescence and chemiluminescence reactions. Enzymes commonly utilized as labels are horseradish peroxidase, alkaline phosphatase, B-galactosidase and glucose oxidase. Although enzymes have an advantage over radioactive labels in that they are very stable and need no special facilities and instrumentation, enzyme immunoassays are generally slower, laborious and less sensitive. Luminescent labels, including fluorescent and chemiluminescent labels, have been utilized as an alternative for radioactive or enzyme labels as they possess the ease of use advantage of radiolabels and the reagent stability advantage of enzymes. Fluorescence detection can be used with a much wider variety of enzymes. However, due to the difficulty of conventional fluorescence detection in discriminating between specific and nonspecific signals and therefore the practical assay detection limit, fluorescence assays lack the sensitivity of either radioactive or enzyme labels, making them seldom the assay method of choice for both research and clinical applications.

Chemiluminescent reactions as label of signal generation are the most sensitive and have been around for decades. Recent advances in DNA technologies have expanded the utilization of these labels as signal generators, but due to the limited number of known reactions that form chemiluminescent products, the luminescence assay method is currently under utilized. Also, luminescent reactions need one or more chemical activation steps, and automation of these reactions is difficult, although needing less complex instrumentation than fluorescence. Even though a large number of luminescence meters viz. luminometers, of various formats and sizes are available, automation of luminescence is complicated and fully-automated luminometers for carrying out binding assays are not available, at least in convenient, small-size analyzers.

The most common luminescence method utilized as a label for signal generation in binding assays is chemiluminescence. This may be classified according to the method utilized for generating the luminescent signal viz. chemiluminescent and bioluminescent labels. Bioluminescence refers to the emission of light by biological molecules and utilizes bioluminescent proteins which can be true enzymes. Examples are luciferases that catalyze the oxidation of luciferin with release of oxyluciferin and emit light, and photoproteins that catalyze the oxidation of luciferin to emit light but do not release the oxidized substrate.

The calcium-sensitive photoproteins, including Aequorin, Obeln, Mnemiopsin, Berovin, Pholasin, Luciferases and photoproteins isolated from Pelagia, Cypridina and ostracods were widely researched and employed in binding assays. Furthermore, the genes of some of them have been cloned, permitting the production of large quantities. Aequorin is the most commonly studied and employed member of this group of calcium-sensitive photoproteins.

Native aequorin, isolated from jellyfish (Aequorea), has been purified and utilized as a label in varieties of monitoring systems. Native aequorin consists of a single polypeptide chain of MW 21,000 Daltons (called apoaequorin), containing one mole each of tightly bound coelenterate luciferin and oxygen. This complex is stable in the absence of calcium ions. Aequorin can also be produced by recombinant DNA techniques, for example as discussed by Cormier, M. J., U.S. Patent 5,162,227 and Zenno. S. et al. in U.S. Patent 5,288,623. Furthermore, modified forms of aequorin with enhanced bioluminescence properties have also been produced by recombinant DNA procedures, as disclosed by Prasher, D. in U.S. Patent 5,360,728.

The mechanism of photon emission of aequorin is well understood. Aequorin has a high-affinity for calcium ions. In the presence of excess calcium ions, aequorin catalyzes the oxidation of luciferin to oxyluciferin in a single turnover event with the generation of a glow-type "flash reaction" which persists for approximately 10 seconds with a relatively high quantum yield. Although peak light emission is initiated upon binding of three moles of calcium ions per mole of aequorin, binding of aequorin with trace of amount of free calcium results in partial oxidation of coelenterazine and yields apoaequorin, coelenteramide, CO and light.

As aequorin can be detected at the attomole level and the wavelength of its luminescence is very narrow and may be detected using commercially available luminometers, luminescence of aequorin offers many advantages including speed, high sensitivity and accuracy with a low background. Therefore, aequorin has proven useful as a label in binding assays. Furthermore, stable conjugates of aequorin with various binding reagents such as receptors, hormones, lectins, antibodies, antigens, DNA, RNA, oligonucleotides, and glycoproteins have been developed and a large number of such conjugates are commercially available.

When utilized in combination with streptavidin, biotinylated derivative of aequorin demonstrates the ability to detect nanogram to subnanogram amounts of the target analyte, including proteins and DNA, immobilized onto the wells of microtiter plates or nitrocellulose membranes. Marketed luminometers that employ aequorin are designed with injectors to inject calcium at a particular moment. Although several clinical testing assays that utilize aequorin have been introduced, the luminometers are not automated and tanks of solutions of calcium have to be included. This makes them awkward to use by non-specialized personnel. A luminescent binding assay that utilizes aequorin and whole blood is disclosed by Pankratz et al in U.S. Patent No. 5,876,935.

In the cell of an organism, calcium (Ca) is an important intracellular second messenger for a wide variety of processes, which have physiological, biochemical and pathophysiological significance such as muscle contraction, neurotransmitter release, ion channel gating and exocytosis. Attempts to understand and measure the rapid changes and release of intracellular calcium have resulted into the introduction of a class of calcium-sensitive compounds called calcium-caging compounds. Calcium-caging compounds have the ability to be loaded with calcium and to unload their calcium upon stimulation. Unloading of the encased calcium may be induced by several methods, one of which is through exposure to light. Light-stimulation release of calcium from the caged compounds (called photolysis) is usually done by illumination for fractions of a second with laser pulses typically in the UV 350-400 nm region of the spectrum. Two different classes of Ca-caging compounds have been introduced; the BAPTA derivative such as the nitr-5 and nitr-7 and the EDTA or EGTA derivatives such as DM-nitrophen and nitrophenyl-EGTA. BAPTA is 1,2-bis(ortho-aminophenoxy)ethane-N,N,N',N'-tetraacetic acid. Nitr-7 is cis-1-(2-bis(carboxymethyl)amino-5-(1-hydroxy-1-(2-nitro-4,5-methylenedioxyphenyl)methyl)phenoxy)-2-(2-bis(carboxymethyl)amino-5-methylphenoxy)cyclopentane. Nitr-5 is 1-[2-Amino-5-(1-hydroxy-1-[2-nitro-4,5-methylenedioxyphenyl]methyl)phenoxy]-2-)2'-amino-5'methylphenoxy)ethane-N,N,N',N'-tetraacetic acid. DM-nitrophen is 1-(4,5 dimethoxy-2-nitrophenyl)-1,2 diaminoethane-N, N, N', N'-tetraacetic acid and nitrophenyl-EGTA i.e. nitrophenyl ethylenebis(oxyethylenenitrilo) tetraacetic acid. The latter class was designed to produce photosensitive derivatives of chelators with known high affinity for calcium, see US Patent 5,446,186 and U.S. Patent 4,981,985. The DM-nitrophen and nitrophenyl-EGTA calcium-caging compounds offer the advantage of calcium-selectivity. On irradiation, the chelated calcium cleaves with the subsequent cleaved remainders having a substantially lower affinity for the released calcium. Thus, large mounts of calcium are rapidly released. These photosensitive calcium-caging compounds are commercially available.

A binding assay e.g. immunoassay or nucleic acid binding assay, that utilizes photosensitive calcium-caging compounds would be useful.

### Summary of the Invention

A method has now been found for conducting a receptor-ligand binding assay utilizing calcium caging compounds and calcium-sensitive luminescent compounds.

Accordingly, one aspect of the present invention provides a method for conducting a binding assay to detect the presence of an analyte in a solution, comprising the steps of:
(a) contacting a first binding partner with said solution, said first binding partner being conjugated to a calcium-sensitive chemiluminescent material;
(b) after a period of time, mobilizing the first binding partner in a predetermined direction along one side of an elongated matrix of a capture strip so as to contact the first binding partner with a stripe transversely located on said capture strip, said transverse stripe having immobilized second binding partner and containing a calcium-caging compound,
(c) allowing a period of time sufficient for the first binding partner to contact said second binding partner immobilized onto said transverse stripe,
(d) exposing said transverse stripe of said capture strip to a pulse of ultraviolet light to effect the release of calcium from the calcium caging compound; and
(e) measuring luminescence emitted by the calcium-sensitive luminescent material.

Another aspect of the present invention provides a method for conducting a binding assay to detect the presence of an analyte in a solution, comprising the steps of:
(a) contacting said solution with a first binding partner of a binding reaction, said first binding partner being immobilized on a solid surface, said solid surface being paramagnetic particles and said first binding partner being conjugated to calcium-sensitive luminescent material;
(b) after a period of time, mobilizing the paramagnetic particles in a predetermined direction along one side of an elongated matrix of a capture strip so as to contact the particles with a stripe of a second binding partner transversely located on said capture strip, said capture strip having the second binding partner immobilized onto said transverse stripe, said transverse stripe additionally containing a calcium-caging compound,
(c) allowing a period of time sufficient for the paramagnetic particles to contact said second binding partner immobilized onto said transverse stripe,
(d) exposing said transverse stripe of said capture strip to a pulse of ultraviolet light to effect the release of calcium from the calcium caging compound; and
(e) measuring luminescence emitted by the calcium-sensitive luminescent material.

In preferred embodiments of the invention, the method is an immunoassay for detecting and quantifying an antigen, an immunoassay for detecting and quantifying an antibody, or a nucleic acid hybridization assay for detection and quantifying a particular sequence of nucleic acid.

In another embodiment, the solution is pretreated prior to contacting the calcium sensitive chemiluminescent material in step (a), especially filtered to remove calcium, the filter containing an agent for removal of calcium.

In another embodiment, the solution is whole blood, said whole blood being pretreated by filtering prior to being contacted with the paramagnetic particles.

In a further embodiment, the luminescent material is calcium-sensitive luminescent material, especially aequorin, Obeln, Mnemiopsin, Berovin, Pholasin, Luciferases or photoproteins isolated from Pelagia, Cypridina and ostracods.

In still further embodiments, the ultraviolet light is in the form of a pulse of light in the range of 250-400 nm, and the luminescence is measured by a photomultiplier. In particular, the calcium-sensitive luminescent material is aequorin and photomultiplier detects light of 400-600 nm and is protected from the magnetic field.

In other embodiments, the elongated capture strip is formed of nitrocellulose, polyacrylamide or other natural or synthetic polymer and has a transverse stripe with immobilized second binding partner and impregnated with a calcium caging compound.

In a further embodiment, the calcium-caging compound is loaded with calcium in excess of the stoichiometric amount for said calcium-sensitive luminescent material. Preferably, the calcium-caging compound is nitr-5, nitr-7, DM-nitrophen or nitrophenyl-EGTA.

A further aspect of the invention provides a method for conducting a binding assay to detect the presence of an analyte in a solution, comprising the steps of:
(a) immobilizing a first binding partner of a binding reaction onto a solid surface, said solid surface being paramagnetic particles, said first binding partner being biotinylated;
(b) contacting said first binding partner with said solution;
(c) contacting the solution with a second binding partner, said second binding partner being conjugated to a calcium-sensitive luminescent material;
(d) after a period of time, mobilizing the paramagnetic particles in a predetermined direction along one side of an elongated matrix of a capture strip so as to contact the particles with a stripe transversely located on said capture strip, said capture strip having streptavidin immobilized onto said transverse stripe, said transverse stripe additionally contain a calcium-caging compound,
(e) allowing a period of time sufficient for the paramagnetic particles to contact said streptavidin immobilized onto said transverse stripe,
(f) exposing said transverse stripe of said capture strip to a pulse of ultraviolet light to effect the release of calcium from the calcium caging compound; and
(g) measuring luminescence emitted by the calcium-sensitive luminescent material.

In an embodiment, steps (b) and (c) are carried out simultaneously.

Yet another aspect of the invention provides a method for conducting a binding assay to detect the presence of an analyte in a solution, comprising the steps of:
(a) contacting a first binding partner with said solution, said first binding partner being biotinylated;
(b) after a period of time, contacting the solution with a second binding partner, said second binding partner being conjugated to a calcium-sensitive luminescent material;
(c) after a further period of time, mobilizing the binding partners in a predetermined direction along one side of an elongated matrix of a capture strip so as to contact the binding partners with a stripe transversely located on said capture strip, said capture strip having streptavidin immobilized onto said transverse stripe, said transverse stripe additionally contain a calcium-caging compound,
(d) allowing a period of time sufficient for the binding partners to contact said streptavidin immobilized onto said transverse stripe,
(e) exposing said transverse stripe of said capture strip to a pulse of ultraviolet light to effect the release of calcium from the calcium caging compound; and
(f) measuring luminescence emitted by the calcium-sensitive luminescent material.

In an embodiment, steps (a) and (b) are carried out simultaneously.

In a further embodiment, the elongated capture strip has a transverse stripe impregnated with streptavidin and a calcium-caging compound.

In a still further aspect, the present invention provides an elongated capture strip for binding assays, said strip having a transverse section thereof impregnated with a binding partner and a caged calcium compound.

In preferred embodiments, the capture strip is formed from nitrocellulose, polyacrylamide, polyamide or any other synthetic or naturally occurring polymer.

In another embodiment, the capture strip is in a housing, especially within a support as a single use testing cartridge.

In a further embodiment, the binding partner is streptavidin.

A further embodiment of the invention provides a plastic cartridge comprising:
a housing with a receptacle for receipt of a sample, a reservoir containing biotinylated first binding partner immobilized onto paramagnetic particles and a second binding partner conjugated to calcium-sensitive chemiluminescence material, an elongated capture strip within the housing and in fluid communication with the reservoir, said capture strip having a transverse section thereof impregnated with streptavidin and a calcium-caging compound, said transverse section being protected with a light barrier.

In a preferred embodiment, there is a filter between the receptacle and the reservoir, especially a filter containing an agent for removal of calcium.

A further embodiment provides apparatus for carrying out a binding assay comprising a housing enclosing (a) a receptacle to receive the said plastic cartridge; (b) a means for removing the light protective layer over the transverse stripe; (c) a electromagnet to provide a magnetic field; (e) a ultraviolet light source to project light on a pre-selected portion of the capture strip, and (f) a photomultiplier disposed to receive light emitted by the pre-selected portion of the capture strip.

### Brief Description of the Drawings

The present invention is illustrated by the embodiment shown in the drawings, in which:
Fig. 1 is a schematic representation of a capture strip of the present invention;
Fig. 2 is a schematic representation of the cartridge of the present invention;
Fig. 3 is a schematic representation of apparatus of the present invention;
Fig. 4 is a graphical representation of photoemission from a sample in Example I;
Fig. 5 is a graphical representation of photoemission from a sample in Example I, after photolysis with ultraviolet light;
Fig. 6 is a graphical representation of the combined graphs of Fig.s 5 and 6;
Fig.s 7 and 8 are graphical representations of photoemission from samples in Example II.

### Detailed Description of the Invention

While the present invention may be used for detection and quantification of a binding partner of a binding reaction, it will be described herein with particular reference to a sandwich immunoassay for the detection and quantification of antigen that additionally employs a biotin-streptavidin reaction and paramagnetic particles, which is preferred.

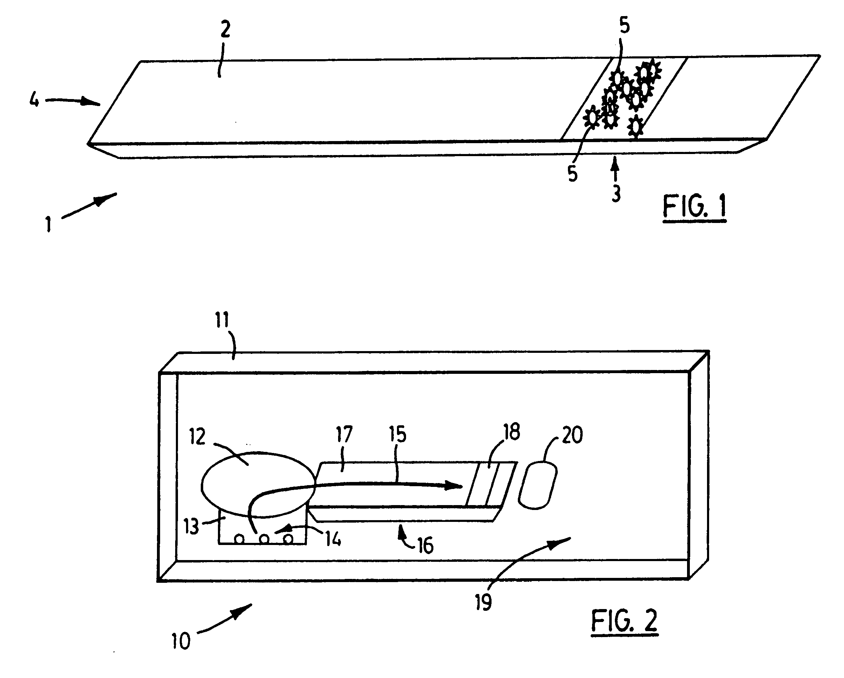

Fig. 1 shows a capture strip, generally indicated by 1. Capture strip 1 has an elongated matrix 2. Elongated matrix 2 is formed from a matrix composition that will permit the paramagnetic particles with associated immune complex thereon to pass along the capture strip under the influence of a magnetic field. Examples of the matrix composition include nitrocellulose, polyacrylamide, polyamide or other synthetic or naturally-occurring polymer. In other embodiments, the matrix is in the form of a microfluidic channel, especially a channel etched into the capture strip. In this embodiment, a matrix composition would not be required. The capture strip must be formed of a clear material, especially in the location of the transverse stripe 3, to permit passage of light. Examples of such materials include acrylic polymers, polystyrene, acrylonitrile-butadiene-styrene (ABS), polycarbonate and other transparent polymers.

Elongated matrix 2 has transverse stripe 3 located towards one end, such end being opposed to inlet end 4. Transverse stripe 3 contains both streptavidin and calcium-loaded calcium caging compounds 5 or such other compounds as are disclosed herein.

Fig. 2 shows a plastic cartridge for carrying out the immunoassay reaction, generally indicated by 10. Plastic cartridge 10 has cartridge housing 11. Cartridge housing 11 has a sample receiving receptacle that contains a filter 12, a reservoir 13 for housing the paramagnetic particles 14 and the second binding partner thereon, and particle path 15. Particle path 15 is in fluid communication with the reservoir 13 and leads from reservoir 13 into capture strip 16, where particle path 15 extends along elongated path 17 of capture strip 16 to transverse stripe 18. Filter 12, reservoir 13 and capture strip 16 are all located within a holder 19 that forms part of plastic cartridge 10. It is to be understood that at least transverse stripe 18 would have a peelable protective light barrier thereon which would be removed before use, i.e. before exposure to light from the light source. Additionally, the elongated capture strip is in communication with a discharge reservoir 20 at the opposite end of the sample receiving receptacle for receiving reagents that pass from the transverse stripe 18.

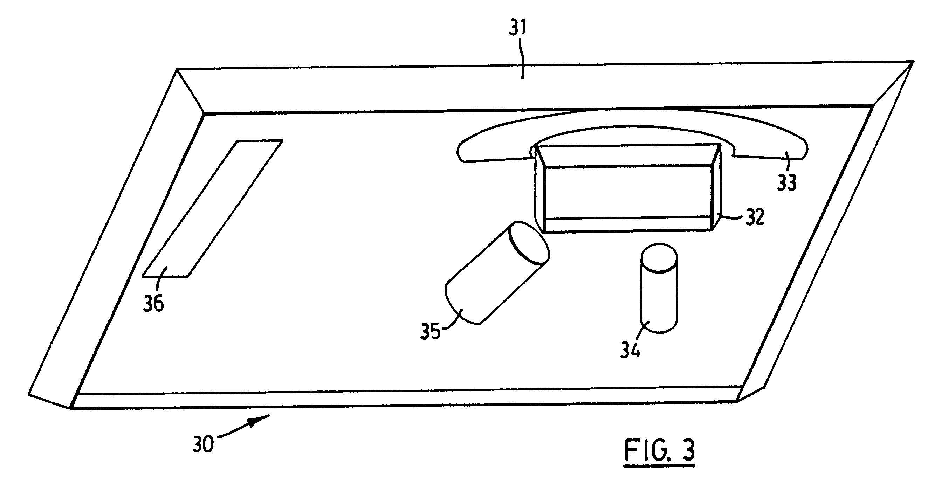

Fig. 3 shows a testing platform apparatus, generally indicated by 30. Testing platform apparatus 30 has housing 31. Within the housing 31 are plastic cartridge 32, electromagnet 33, ultraviolet light source 34 and photomultiplier 35. Plastic cartridge 32 has been described previously, and could be accommodated within the receptacle of the housing 31 of the platform apparatus 30. Electromagnet 33 extends for the length of plastic cartridge 32. Electromagnet 33 is preferably comprised of sectional pre-determined magnetic fields that facilitate mobilization of paramagnetic beads (particles) along the elongated path 17 into capture strip 16. Ultraviolet light source 34 is directed at plastic cartridge 32 and, in particular, at transverse stripe 18 of plastic cartridge 32, which has been described previously. Photomultiplier 35 is also directed at transverse stripe 18.

Testing platform apparatus 30 additionally has display 36, which would typically be an LCD display. Housing 31 would also contain appropriate controls and associated computer hardware and software to permit appropriate interpretation of the results obtained.

In use, a sample containing an antigen e.g. blood, is placed on filter 12. Liquid containing the target analyte passes through filter 12 into reservoir 13, where it contacts the paramagnetic particles which has the biotinylated first binding partner (capture partner) immobilized onto it and the second binding partner conjugated to a calcium-sensitive luminescent label (detector partner). In addition, it is understood that when the cartridge is designed to detect and quantify an antigen, the first and second binding partners are antibodies. On the other hand, when the cartridge is designed for detecting an antibody, the first binding partner is an antigen while the second binding partner is an antibody.

The plastic cartridge 10, 32 is then placed in the testing platform 30 if it is not already located within the platform. It is understood that at least transverse stripe 18 of capture strip 16 of plastic cartridge 10 would need to be protected from light. Such protection could be removed within testing platform 30, in a light-tight manner. Such removal could be automatic.

After allowing appropriate time for the binding reaction, the magnetic field is applied, using electromagnet 33. Then, the paramagnetic particles and attached immune complexes move along particle path 15 and into capture strip 16. The particles then pass along capture strip 16 until transverse stripe 18 is reached. At that time, the particles become bound to streptavidin, already located in transverse stripe 18, through the biotinylated binding partner immobilized onto the particles. Transverse stripe 18 additionally contains a calcium caging compound.

After an appropriate time, which would depend in particular on the dimensions of the capture strip 16, but which conveniently could be 4-6 minutes, ultraviolet light source 34 is activated and sends a pulse of light onto transverse stripe 18. The light causes the release of calcium from the calcium-loaded calcium caging compound, which occurs essentially instantaneously. The calcium contacts the calcium-sensitive chemiluminescent material, which then glows for a short period of time in the range of 4-10 seconds. The light that is emitted is detected by the photomultiplier 35, and the amount of light emitted is interpreted and is displayed on display 36. The rate of emission of light depends on the energy of the ultraviolet light source. High energy levels will cause a high emission rate i.e. a sharp peak of emitted light, but it is preferred that lower energy levels be used such that the emitted light is a broader band. This will lead to more accurate recording of the amount of light by the photomultiplier, especially if emission of light commences prior to completion of the re-setting of the photomultiplier to its zero or null point, as discussed herein.

Some examples of the source of the solution containing or suspected of containing the target analyte that is subjected to the method of the present invention are blood or blood products, saliva, or any other body fluids. Other solutions could be tested.

Utilizing calcium-sensitive luminescent material as the signal generating label in binding assays requires that the solutions that will be contacting the calcium-sensitive luminescent conjugate have to be calcium-free before the moment of generating the light emission. Calcium in the solution will react with the calcium-sensitive luminescent conjugate. In particular, the solution should contain less than 20 nanomolar of calcium. Furthermore, when the goal is to determine the presence of an analyte in whole blood, the sample of blood normally must be pretreated to remove cellular components and hemoglobin, which can interfere with the specific signal of the binding assay. Filters impregnated in calcium-chelating agents would achieve both functions of removing the cellular components as well as calcium from the solutions that contain the target analyte.

The method of the invention disclosed herein utilizes any calcium-sensitive luminescent material for the signal generation in binding assays including, but not limited to, aequorin, mitrocomin, clytin, obelin, mnemiopsin, berovin, halistaurin and phialidin. In case of utilizing a calcium-sensitive luminescent photoprotein, other than aequorin, the optimal wavelength, other than 469 nm that is the optimal wavelength for detecting the aequorin signal, of the photomultiplier has to be adapted accordingly. For example, the wavelength may be 400-600 nm.

Photolysis of the calcium caging compounds may be achieved by many light sources generating light within a wavelength from 250-400 nm. Such light sources are referred to herein as ultraviolet light sources. One such source is a laser source, which is a convenient source to accurately deliver light for less than 1 millisecond at a wavelength of 300-350 nm. Upon the release of calcium from the caging compound when light-triggered and upon binding of three moles of calcium ions per mole of aequorin, the light emission is initiated with a flash of blue light that persists for approximately ten seconds. The generated light could then be measured with a suitable photomultiplier both as peak light or total photon counting.

The method of the present invention preferably utilizes a time-resolved mechanism, particularly time-resolved chemiluminescence. In this method, there is a short period of time between the flash emitted by the ultraviolet light source and the emission of light by the calcium-sensitive luminescent material. The calcium-sensitive luminescent material is selected to obtain such a period of time. The photomultiplier records the stray light after the flash from the ultraviolet source followed by a period of zero or substantially zero light, which is then followed by the emission of light. During the period of zero light, the reading on the photomultiplier can be re-set to its zero or null point, thereby permitting a more accurate reading of the emission of light. The period between the pulse of light and the emission of light is short, but such time is sufficient to reset the photomultiplier to a zero baseline.

The ultraviolet light source should be generally shielded from the capture strip, with the light being focussed on the transverse stripe, e.g. using coated quartz lenses. As discussed herein, the intensity of the light source may be varied, but one embodiment is at least 150 mJ.

Native calcium-sensitive luminescent photoproteins are particularly useful as a label in the method of the present invention of carrying out binding assays. Other modified recombinant DNA-driven forms of these photoproteins with enhanced luminescence, due to either the ability of regeneration or a higher affinity for calcium, are also compatible with the method of the invention.

Although encasing compounds such as in light sensitive liposomes have been extensively researched, the recent introduction of cation-specific caging compounds is particularly useful in carrying out the method of the invention. The recently introduced two classes of calcium-caging compounds which are derivatives of chelating agents are particularly useful as they are more stable and the mechanism(s) of their triggering is well defined. In particular, the breakdown derivatives of DM-nitrophen derivative of EDTA (ethylenedinitrilo tetraacetic acid, disodium salt) and nitrophenyl-EGTA (ethylenebis(oxyethylenenitrilo) tetraacetic acid) have a very low affinity for calcium once light-triggered. Also, the wavelength of fluoroescence of the cleaved compounds is much different than that of the calcium-sensitive luminescent photoproteins and lasts for a very brief period of time. These photosensitive calcium-caging compounds are commercially available.

The detector materials on the transverse stripe may be located and immobilized on glass beads, which provides a high surface area of detector material.

Combining aequorin, which can be detected at the attomol level, together with exploiting the high affinity of biotin/streptavidin reaction offers a very high sensitivity of the method of the invention to measure analytes at a subnanogram level of detection. Furthermore, modified forms of streptavidin are also compatible with the method of the invention and both streptavidin and its derivatives could be easily immobilized onto the lateral transverse stripe of the capture matrix strip.

According to the method of the invention for carrying out a binding assay, separation of the bound from free luminescent label is effected by applying a magnetic field. It will be recognized that the force on suspended magnetic particle subjected to a magnetic field urges the particle to move to stronger field regions, typically towards the pole of a magnet, and that the strength of the force depends both on the field gradient and magnetism induced in the particle by the field. Thus, for rapid separation, a strong separator and a highly magnetizable particle appear preferable. Furthermore, the electromagnet is capable of producing several field gradients in pre-determined optimized directions.

Microscopic magnetic particles ranging from 0.7-1.5 microns are compatible with the method of the invention and may be used as they provide a large surface area for coating with proteins, for example, those disclosed in U.S. Patents No.s 3,970,518; 4,018,886; 4,230,685; 4,267,234; 4,452,773; 4,554,088; and 4,659,678. However, smaller size paramagnetic particles of the size 0.03 to 10, especially 0.5-1.0 micrometers, as described in the US Patent No. 5,736,349 are more suitable as large size particles of magnetic material tend to adhere to one another after removal of the magnetic field, due to residual magnetism. Suitable magnetic materials include ferromagnetic, ferrimagnetic and superparamagnetic materials. Other suitable magnetic materials include oxides, such as, for example, ferrites, perovskites, chromites and magnetoplumbites. Nickel particles may also be used.

The magnetic separation apparatus/method used for separating of target analyte-bearing magnetic particles from test media will depend on the nature and size of the magnetic particle. The micron-size magnetic particles suitable in the invention are readily removed from solution by means of commercially available magnetic separation devices. These devices employ a single relatively inexpensive permanent magnet located external to a container holding the test medium. Examples of such magnetic separators are the MAIA Magnetic Separator manufactured by Serono Diagnostics, Norwell, Mass., the DYNAL MPC-1 manufactured by DYNAL, Inc., Great Neck, N.Y. and the BioMag Separator, manufactured by Advanced Magnetics, Inc., Cambridge, Mass. The preferred magnetic separator for the present invention would have several aligned field gradients. In particular, multiple magnets could be used to effect stirring of the paramagnetic particles, and then to successively move the particles out of the vessel onto and along the capture strip. The magnets could be operated independently and/or in a coordinated sequence so as to effect stirring and then the movement of the particles along a pre-determined path e.g. to the capture strip and then to the transverse stripe. An example of the use of magnets in the stirring of magnetic particles is disclosed in US Patent No. 5,835,329.

In developing a bioassay, there are many considerations for the assay to attain value in the clinical laboratory. One consideration is the signal response to changes in the concentration of analyte. A second consideration is the ease with which the protocol for the assay may be carried out. A third consideration is the variation in interference from sample to sample. Also, ease of preparation and purification of the reagents, availability of equipment, ease of automation and interaction with material of interest are some of the additional considerations in developing a useful assay.

The method of the invention for carrying out a binding assay offers improvement in such consideration. The invention offers the high sensitivity of luminescence, the availability, sensitivity and high quantum yield of calcium-sensitive luminescent material, particularly aequorin, the physical characteristic of calcium-sensitive luminescent material to response to changes in calcium without having to manually inject calcium, the availability of commercial luminometers with photomultipliers that could detect the generated photons without the interference of the magnetic field, and the development of solid chromatographic capturing matrices that offer the convenience of point of care testing. Most important, the large difference in the wave length of exciting the caged calcium (240-400 nm) and the wavelength of measuring the generated photons (450-500 nm) facilitates detection of emitted light without interference from the incident light from the ultraviolet light source or due to fluorescence of the medium. Thus, a time-resolved chemiluminescence is used, as described herein.

The present invention of carrying receptor-ligand binding reaction utilizing a calcium-sensitive chemiluminescent label has been described herein with reference to the paramagnetic particle having the biotinylated first binding partner immobilized onto its surface, the chemiluminescent material conjugated to the second binding partner, and with the calcium caging compound being associated with the streptavidin to carry out a full sandwich immunoassay for detecting and quantifying an antigen as the preferred embodiment of the method invention. However, it is to be understood that the method of the invention is as equally beneficial in detecting an antibody as well as a nucleic acid as the target analyte of a receptor-ligand binding reaction. Also, it is to be understood that the method of the invention could be carried out with the first binding partner conjugated to a calcium-sensitive luminescent material and immobilized onto paramagnetic particles and the second binding partner immobilized in the transverse stripe of the capture strip together with the calcium caging compound.

The present invention is illustrated by the following examples.

### EXAMPLE I

5 µg of aequorin in 10 µl was added to a 200 µl solution of buffered 1-(4,5 dimethoxy-2-nitrophenyl)-1,2 diaminoethane-N, N, N', N'-tetraacetic acid (DM-NP) containing calcium chloride. The solution contained 80 mM of 4-morpholine propane sulphonic acid (MOPS) buffer and 20 mM of KCI, with the pH of the solution adjusted to 7.2. The DM-NP was loaded with calcium up to 75% i.e. 2mM DM_NP + 1.5 mM CaCl₂.

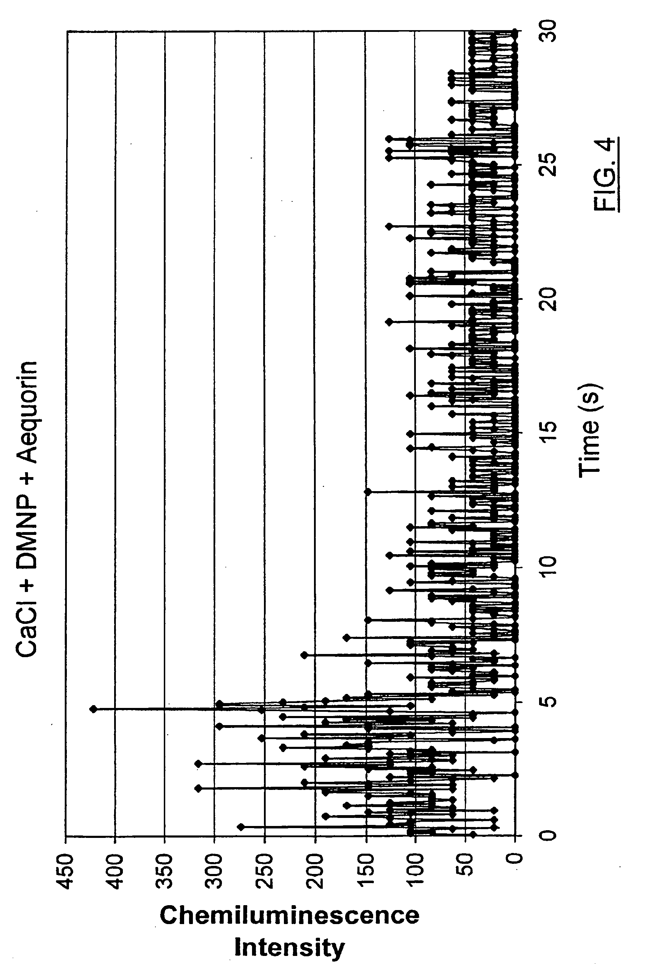

Photoemission from the solution was monitored for 30 seconds at a wavelength of 470 nm. The results obtained shown in Fig. 4, shown minimal emission. The solution was then photolysed using an ultraviolet light of a wavelength of 347 nm. The pulse of light was 100 mJ.

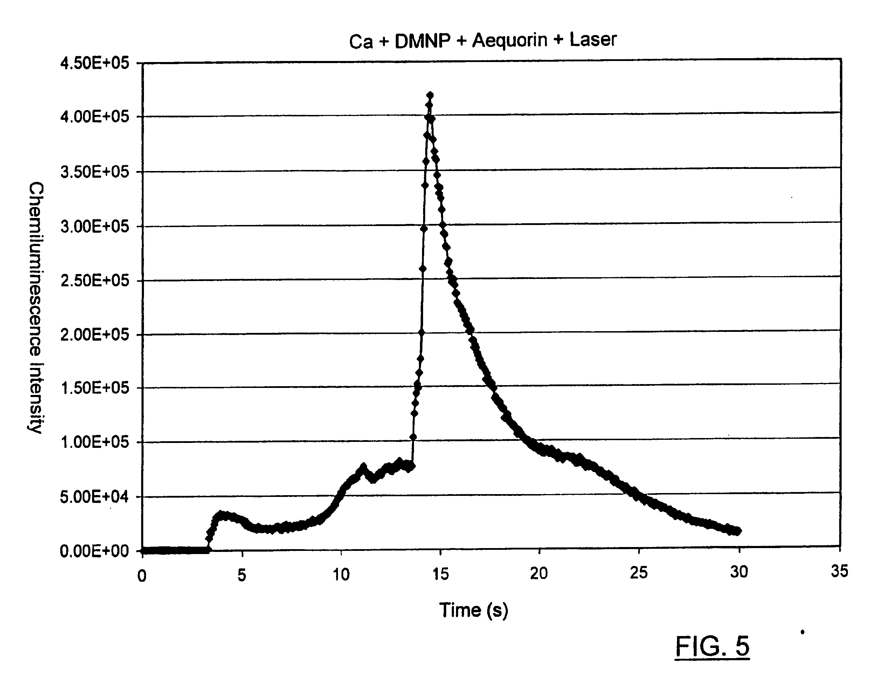

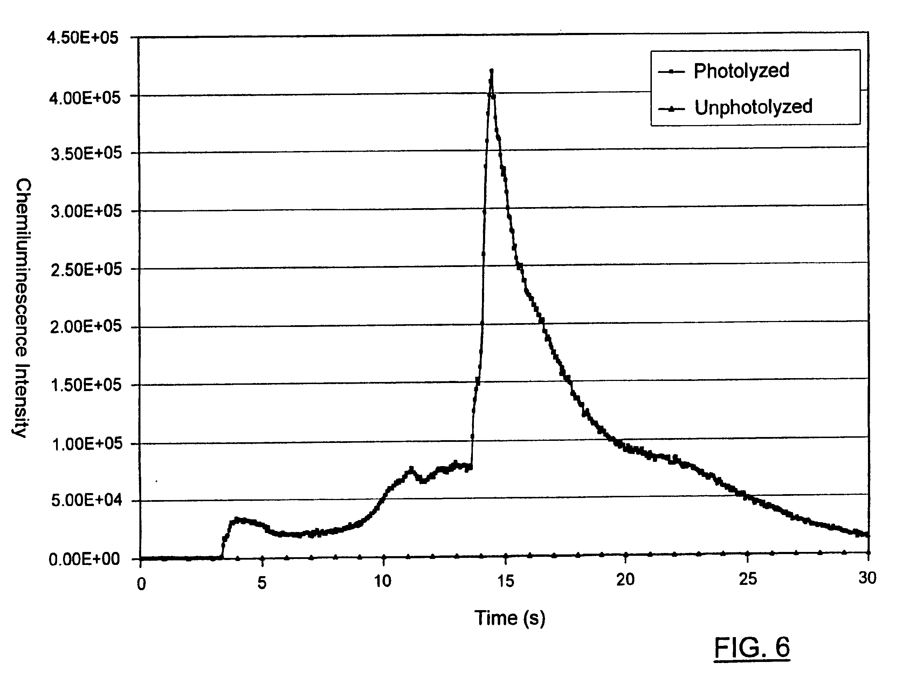

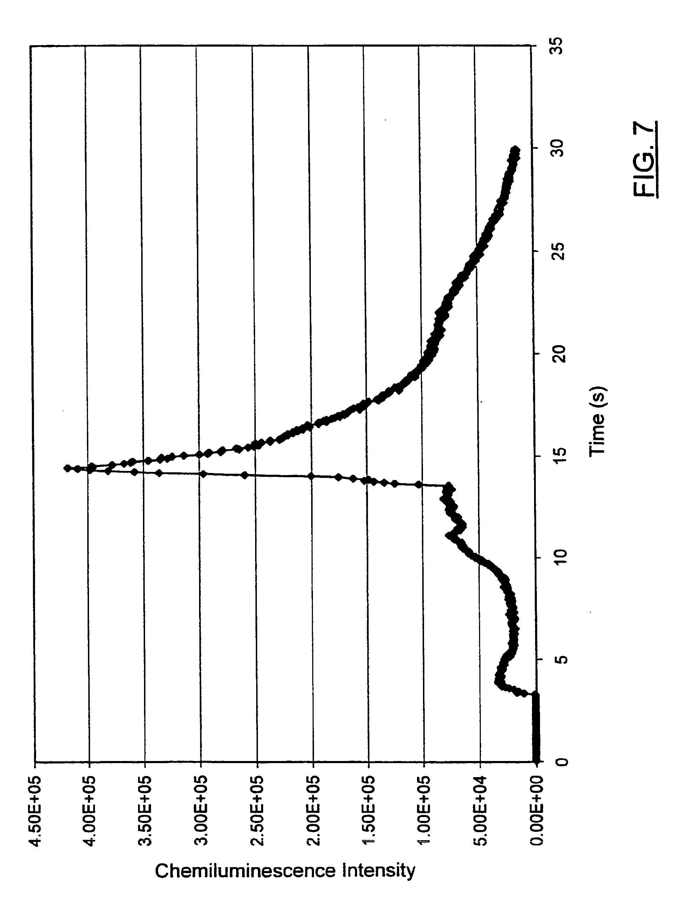

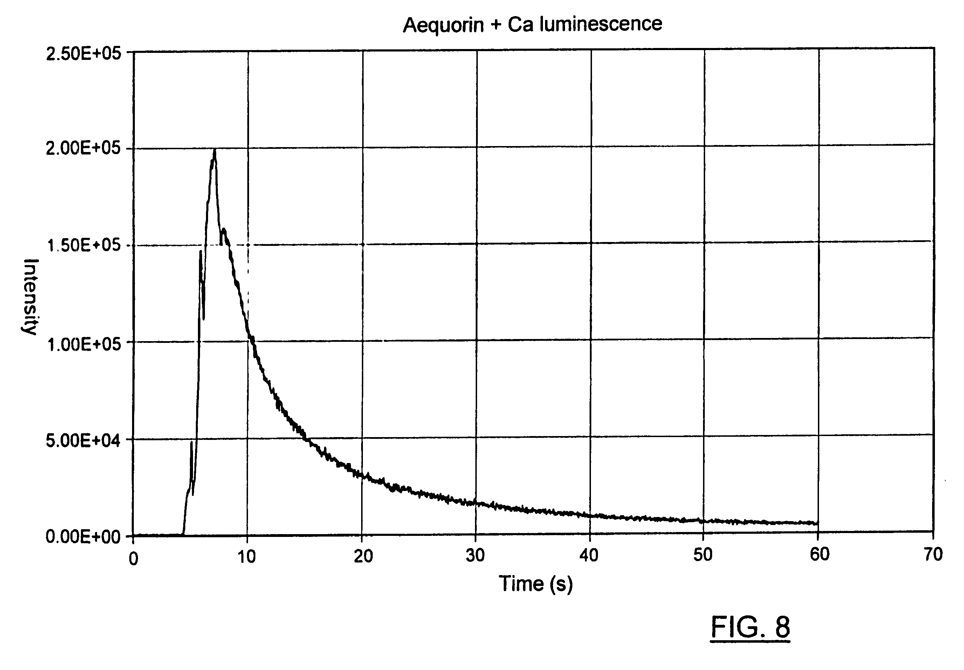

The results obtained are shown in Fig. 5, and the results of Fig.s 4 and 5 are combined in Fig. 6.

The results show that the pulse of ultraviolet light caused release of sufficient calcium to trigger photoemission from aequorin. Prior to the pulse of ultraviolet light (Fig. 4), the caged calcium did not trigger emission from aequorin. Emission of light was complete within 30 seconds.

### EXAMPLE II

The procedure of Example I was repeated using solutions of 5 µg of aequorin. In separate experiments, 1 mM of CaCl₂ and 500 µM of CaCl₂ were added. The total photon count after the pulse of ultraviolet light was monitored at 470 nm for 30 seconds.

The results are shown in Fig.s 7 and 8, respectively. Although the peak heights are different, the total amount of photons emitted is the same. Thus, the total amount of photons can be used to monitor the reaction. Under more controlled conditions i.e. not the manual addition of these examples, peak intensity could also be used.

## Claims

1. A method for conducting a binding assay to detect the presence of an analyte in a solution, comprising the steps of:
(a) contacting a first binding partner with said solution, said first binding partner being conjugated to a calcium-sensitive chemiluminescent material;
(b) after a period of time, mobilizing the first binding partner in a predetermined direction along one side of an elongated matrix of a capture strip so as to contact the first binding partner with a stripe transversely located on said capture strip, said transverse stripe having immobilized second binding partner and containing a calcium-caging compound,
(c) allowing a period of time sufficient for the first binding partner to contact said second binding partner immobilized onto said transverse stripe,
(d) exposing said transverse stripe of said capture strip to a pulse of ultraviolet light to effect the release of calcium from the caged calcium compound; and
(e) measuring luminescence emitted by the calcium-sensitive luminescent material.

2. The method of Claim 1 for conducting a binding assay to detect the presence of an analyte in a solution, comprising the steps of:
(a) contacting said solution with a first binding partner of a binding reaction, said first binding partner being immobilized on a solid surface, said solid surface being paramagnetic particles and said first binding partner being conjugated to calcium-sensitive luminescent material;
(b) after a period of time, mobilizing the paramagnetic particles in a predetermined direction along one side of an elongated matrix of a capture strip so as to contact the particles with a stripe of a second binding partner transversely located on said capture strip, said capture strip having the second binding partner immobilized onto said transverse stripe, said transverse stripe additionally containing a calcium-caging compound,
(c) allowing a period of time sufficient for the paramagnetic particles to contact said second binding partner immobilized onto said transverse stripe,
(d) exposing said transverse stripe of said capture strip to a pulse of ultraviolet light to effect the release of calcium from the calcium caging compound; and
(e) measuring luminescence emitted by the calcium-sensitive luminescent material.

3. The method of Claim 2 in which the method is an immunoassay for detecting and quantifying an antigen, an immunoassay for detecting and quantifying an antibody, or a nucleic acid hybridization assay for detection and quantifying a particular sequence of nucleic acid.

4. The method of any one of Claims 1-3 in which the solution is pretreated prior to contacting the calcium sensitive luminescent material in step (a).

5. The method of Claim 4 in which the solution is filtered to remove calcium, the filter containing an agent for removal of calcium.

6. The method of any one of Claims 1-5 in which the solution is whole blood, said whole blood being pretreated by filtering prior to being contacted with the calcium sensitive luminescent material.

7. The method of any one of Claims 1-6 in which the calcium-sensitive luminescent material is aequorin, Obeln, Mnemiopsin, Berovin, Pholasin, Luciferases or photoproteins isolated from Pelagia, Cypridina and ostracods.

8. The method of any one of Claims 1-7 in which the ultraviolet light is in the form of a pulse of light in the range of 250-400 nm, and the luminescence is measured by a photomultiplier.

9. The method of Claim 8 in which the calcium-sensitive luminescent material is aequorin and in which the photomultiplier detects light of 400-600 nm and is protected from the magnetic field.

10. The method of any one of Claims 1-9 in which the elongated capture strip is formed of nitrocellulose, polyacrylamide or any other natural or synthetic polymer.

11. The method of Claim 10 in which the elongated capture strip has a transverse stripe with immobilized second binding partner and impregnated with a calcium caging compound.

12. The method of any one of Claims 1-11 in which the calcium caging compound is loaded with calcium in excess of the stoichiometric amount for said calcium-sensitive luminescent material.

13. The method of any one of Claims 1-12 in which the calcium-caging compound is selected from the group consisting of cis-1-(2-bis(carboxymethyl)amino-5-(1-hydroxy-1-(2-nitro-4,5-methylenedioxyphenyl)methyl)phenoxy)-2-(2-bis(carboxymethyl)amino-5-methylphenoxy)cyclopentane, 1-[2-Amino-5-(1-hydroxy-1-[2-nitro-4,5-methylenedioxyphenyl]methyl)phenoxy]-2-)2'-amino-5'methylphenoxy)ethane-N,N,N',N'-tetraacetic acid, 1-(4,5 dimethoxy-2-nitrophenyl)-1,2 diaminoethane-N, N, N', N'-tetraacetic acid and nitrophenyl-ethylenebis(oxyethylenenitrilo) tetraacetic acid.

14. The method of any one of Claims 1-13 which is an immunoassay for detecting and quantifying an antigen.

15. The method of any one of Claims 1-13 which is an immunoassay for detecting and quantifying an antibody.

16. The method of any one of Claims 1-13 in which the binding assay is nucleic acid hybridization assay for detection and quantifying a particular sequence of nucleic acid.

17. The method of any one of Claims 1-16 in which the calcium-sensitive luminescent material is aequorin.

18. The method of any one of Claims 1-17 in which the ultraviolet light source emits a pulse of light in the range of 250-400 nm.

19. The method of any one of Claims 1-18 in which the luminescence is measured by a photomultiplier.

20. The method of any one of Claims 1-19 in which the calcium-sensitive luminescent material is aequorin and photomultiplier detects light of 400-600 nm and is protected from the magnetic field.

21. A method for conducting a binding assay to detect the presence of an analyte in a solution, comprising the steps of:
(a) immobilizing a first binding partner of a binding reaction onto a solid surface, said solid surface being paramagnetic particles, said first binding partner being biotinylated;
(b) contacting said first binding partner with said solution;
(c) contacting the solution with a second binding partner, said second binding partner being conjugated to a calcium-sensitive luminescent material;
(d) after a period of time, mobilizing the paramagnetic particles in a predetermined direction along one side of an elongated matrix of a capture strip so as to contact the particles with a stripe transversely located on said capture strip, said capture strip having streptavidin immobilized onto said transverse stripe, said transverse stripe additionally contain a calcium-caging compound,
(e) allowing a period of time sufficient for the paramagnetic particles to contact said streptavidin immobilized onto said transverse stripe,
(f) exposing said transverse stripe of said capture strip to a pulse of ultraviolet light to effect the release of calcium from the calcium caging compound; and
(g) measuring luminescence emitted by the calcium-sensitive luminescent material.

22. The method of Claim 21 in which steps (b) and (c) are carried out simultaneously.

23. A method for conducting a binding assay to detect the presence of an analyte in a solution, comprising the steps of:
(a) contacting a first binding partner with said solution, said first binding partner being biotinylated;
(b) after a period of time, contacting the solution with a second binding partner, said second binding partner being conjugated to a calcium-sensitive luminescent material;
(c) after a further period of time, mobilizing the binding partners in a predetermined direction along one side of an elongated matrix of a capture strip so as to contact the binding partners with a stripe transversely located on said capture strip, said capture strip having streptavidin immobilized onto said transverse stripe, said transverse stripe additionally contain a calcium-caging compound,
(d) allowing a period of time sufficient for the binding partners to contact said streptavidin immobilized onto said transverse stripe,
(e) exposing said transverse stripe of said capture strip to a pulse of ultraviolet light to effect the release of calcium from the calcium caging compound; and
(f) measuring luminescence emitted by the calcium-sensitive luminescent material.

24. The method of Claim 23 in which steps (a) and (b) are carried out simultaneously.

25. The method of any one of Claims 21-24 in which the elongated capturing strip has a transverse section thereof impregnated with streptavidin and a calcium-caging compound.

26. The method of any one of Claims 1-25 in which the pulse of ultraviolet light and the detection of chemiluminescence are conducted in a time-resolved manner.

27. The method of any one of Claims 1-26 in which the solution contain less than 20 nanomolar of calcium before the pulse of ultraviolet light.

28. An elongated capture strip for binding assays, said strip having a transverse section thereof impregnated with streptavidin and a calcium caging compound.

29. The elongated capture strip of Claim 28 in which the capture strip is formed from nitrocellulose, polyacrylamide, polyamide or any other synthetic or naturally occurring polymer.

30. The elongated capture strip of Claim 28 or Claim 29 in which the capture strip is in a housing.

31. The elongated capture strip of Claim 30 in which the capture strip is housed within a support as a single use testing cartridge.

32. The elongated capture strip of any one of Claims 28-31 in which calcium-caging compound is selected from the group consisting of cis-1-(2-bis(carboxymethyl)amino-5-(1-hydroxy-1-(2-nitro-4,5-methylenedioxyphenyl)methyl)phenoxy)-2-(2-bis(carboxymethyl)amino-5-methylphenoxy)cyclopentane, 1-[2-Amino-5-(1-hydroxy-1-[2-nitro-4,5-methylenedioxyphenyl]methyl)phenoxy]-2-)2'-amino-5'methylphenoxy)ethane-N,N,N',N'-tetraacetic acid, 1-(4,5 dimethoxy-2-nitrophenyl)-1,2 diaminoethane-N, N, N', N'-tetraacetic acid and nitrophenyl-ethylenebis(oxyethylenenitrilo) tetraacetic acid.

33. A plastic cartridge for conducting a binding assay to detect the presence of an analyte in a solution, comprising:
a housing with a receptacle for receipt of a sample, a reservoir containing biotinylated first binding partner immobilized onto paramagnetic particles and a second binding partner conjugated to calcium-sensitive chemiluminescence material, an elongated capture strip within the housing and in fluid communication with the reservoir, said capture strip having a transverse section thereof impregnated with a calcium-caging compound and streptavidin, said transverse section being protected with a light barrier.

34. The plastic cartridge of Claim 33 in which there is a filter between the receptacle and the reservoir.

35. The plastic cartridge of Claim 33 in which there is a filter containing an agent for removal of calcium.

36. The plastic cartridge of any one of Claims 33-35 in which the calcium-sensitive luminescent material is aequorin, Obeln, Mnemiopsin, Berovin, Pholasin, Luciferases or photoproteins isolated from Pelagia, Cypridina and ostracods.

37. Apparatus for carrying out a binding assay comprising a housing enclosing (a) a receptacle to receive the plastic cartridge of any one of Claims 33-36; (b) a means for removing the light protective layer over the transverse stripe; (c) an electromagnet to provide a magnetic field; (e) an ultraviolet light source to project light on a pre-selected portion of the capture strip, and (f) a photomultiplier disposed to receive light emitted by the pre-selected portion of the capture strip.

38. The apparatus of Claim 37 in which the electromagnet projects multiple magnetic fields along the plastic cartridge.

39. The apparatus of Claim 37 or Claim 38 in which the ultraviolet light source provides light in the range of 250-400 nm.

40. The apparatus of any one of Claims 37-39 in which the photomultiplier detects light in the range of 400-600 nm.

## Patentansprüche

1. Verfahren zum Durchführen eines Bindungstests, um das Vorliegen eines Analyten in einer Lösung zu detektieren, wobei das Verfahren die folgenden Stufen umfaßt:
(a) Inkontaktbringen eines ersten Bindungspartners mit der Lösung, wobei der erste Bindungspartner an ein Calcium-sensitives chemilumineszentes Material konjugiert ist,
(b) nach einer Zeitdauer Mobilisieren des ersten Bindungspartners in einer vorbestimmten Richtung entlang einer Seite einer länglichen Matrix eines Einfangstreifens, um den ersten Bindungspartner mit einem in Querrichtung auf dem Einfangstreifen angeordneten Streifen in Kontakt zu bringen, wobei der Querstreifen einen immobilisierten zweiten Bindungspartner aufweist und eine Calcium einschließende Verbindung enthält,
(c) Zulassen einer Zeitdauer, die ausreichend ist, damit der erste Bindungspartner mit dem zweiten Bindungspartner, der auf dem Querstreifen immobilisiert ist, in Kontakt treten kann,
(d) Aussetzen des Querstreifens des Einfangstreifens an einen Puls von ultraviolettem Licht, um die Freisetzung von Calcium aus der eingeschlossenen Calciumverbindung zu bewirken, und
(e) Messen der von dem Calcium-sensitiven lumineszenten Material emittierten Lumineszenz.

2. Verfahren nach Anspruch 1 zum Durchführen eines Bindungstests, um das Vorliegen eines Analyten in einer Lösung zu detektieren, wobei das Verfahren die folgenden Stufen umfaßt:
(a) Inkontaktbringen der Lösung mit einem ersten Bindungspartner einer Bindungsreaktion, wobei der erste Bindungspartner auf einer festen Oberfläche immobilisiert ist, wobei die feste Oberfläche aus paramagnetischen Teilchen besteht, und der erste Bindungspartner an Calcium-sensitives lumineszentes Material konjugiert ist,
(b) nach einer Zeitdauer Mobilisieren der paramagnetischen Teilchen in einer vorbestimmten Richtung entlang einer Seite einer länglichen Matrix eines Einfangstreifens, um die Teilchen mit einem Streifen eines zweiten Bindungspartners, der in Querrichtung auf dem Einfangstreifen angeordnet ist, in Kontakt zu bringen, wobei der Einfangstreifen den zweiten Bindungspartner auf dem Querstreifen immobilisiert aufweist, wobei der Querstreifen zusätzlich eine Calcium einschließende Verbindung enthält,
(c) Zulassen einer Zeitdauer, die ausreichend ist, damit die paramagnetischen Teilchen mit dem zweiten Bindungspartner, der auf dem Querstreifen immobilisiert ist, in Kontakt treten,
(d) Aussetzen des Querstreifens des Einfangstreifens an einen Puls von ultraviolettem Licht, um die Freisetzung von Calcium aus der Calcium einschließenden Verbindung zu bewirken, und
(e) Messen der von dem Calcium-sensitiven lumineszenten Material emittierten Lumineszenz.

3. Verfahren nach Anspruch 2, wobei das Verfahren ein Immuntest zum Detektieren und Quantifizieren eines Antigens, ein Immuntest zum Detektieren und Quantifizieren eines Antikörpers oder ein Nukleinsäure-Hybridisierungstest zum Detektieren und Quantifizieren einer bestimmten Nukleinsäuresequenz ist.

4. Verfahren nach einem der Ansprüche 1 bis 3, wobei die Lösung vor dem Inkontaktbringen mit dem Calcium-sensitiven lumineszenten Material in Stufe (a) vorbehandelt wird.

5. Verfahren nach Anspruch 4, wobei die Lösung filtriert wird, um Calcium zu entfemen, und der Filter ein Mittel zum Entfernen von Calcium enthält.

6. Verfahren nach einem der Ansprüche 1 bis 5, wobei die Lösung Vollblut ist, wobei das Vollblut vor dem Inkontaktbringen mit dem Calcium-sensitiven lumineszenten Material durch Filtrieren vorbehandelt wird.

7. Verfahren nach einem der Ansprüche 1 bis 6, wobei das Calcium-sensitive lumineszente Material Aequorin, Obeln, Mnemiopsin, Berovin, Pholasin, Luciferasen oder Photoproteine, isoliert aus Pelagia, Cypridina und Ostracoda, ist.

8. Verfahren nach einem der Ansprüche 1 bis 7, wobei das ultraviolette Licht die Form eines Pulses von Licht im Bereich von 250-400 nm hat und die Lumineszenz durch einen Photomultiplier gemessen wird.

9. Verfahren nach Anspruch 8, wobei das Calcium-sensitive lumineszente Material Aequorin ist und der Photomultiplier Licht von 400-600 nm detektiert und von dem magnetischen Feld abgeschirmt ist.

10. Verfahren nach einem der Ansprüche 1 bis 9, wobei der längliche Einfangstreifen aus Nitrozellulose, Polyacrylamid oder irgendeinem anderen natürlichen oder synthetischen Polymer gebildet ist.

11. Verfahren nach Anspruch 10, wobei der längliche Einfangstreifen einen Querstreifen mit einem immobilisierten zweiten Bindungspartner aufweist und mit einer Calcium einschließenden Verbindung imprägniert ist.

12. Verfahren nach einem der Ansprüche 1 bis 11, wobei die Calcium einschließende Verbindung mit einem Überschuß an Calcium gegenüber der stöchiometrischen Menge für das Calcium-sensitive lumineszente Material beladen ist.

13. Verfahren nach einem der Ansprüche 1 bis 12, wobei die Calcium einschließende Verbindung aus der Gruppe ausgewählt ist, bestehend aus cis-1-(2-bis-(Carboxymethyl)-amino-5-(1-hydroxy-1-(2-nitro-4,5-methylendioxyphenyl)-methyl)-phenoxy)-2-(2-bis-(carboxymethyl)-amino-5-methylphenoxy)-cyclopentan, 1-[2-Amino-5-(1-hydroxy-1-[2-nitro-4,5-methylendioxyphenyl]-methyl)-phenoxy]-2-(2'-amino-5'-methylphenoxy)-ethan-N,N,N',N'-tetraessigsäure, 1-(4,5-Dimethoxy-2-nitrophenyl)-1,2-diaminoethan-N,N,N',N'-tetraessigsäure und Nitrophenylethylenbis-(oxyethylennitrilo)-tetraessigsäure.

14. Verfahren nach einem der Ansprüche 1 bis 13, welches ein Immuntest zum Detektieren und Quantifizieren eines Antigens ist.

15. Verfahren nach einem der Ansprüche 1 bis 13, welches ein Immuntest zum Detektieren und Quantifizieren eines Antikörpers ist.

16. Verfahren nach einem der Ansprüche 1 bis 13, wobei der Bindungstest ein Nukleinsäure-Hybridisierungstest zum Detektieren und Quantifizieren einer bestimmten Nukleinsäuresequenz ist.

17. Verfahren nach einem der Ansprüche 1 bis 16, wobei das Calcium-sensitive lumineszente Material Aequorin ist.

18. Verfahren nach einem der Ansprüche 1 bis 17, wobei die Ultraviolettlichtquelle einen Lichtpuls im Bereich von 250-400 nm emittiert.

19. Verfahren nach einem der Ansprüche 1 bis 18, wobei die Lumineszenz durch einen Photomultiplier gemessen wird.

20. Verfahren nach einem der Ansprüche 1 bis 19, wobei das Calcium-sensitive lumineszente Material Aequorin ist und der Photomultiplier Licht mit 400-600 nm detektiert und von dem magnetischen Feld abgeschirmt ist.

21. Verfahren zum Durchführen eines Bindungstests zum Detektieren des Vorliegens eines Analyten in einer Lösung, welches die folgenden Stufen umfaßt:
(a) Immobilisieren eines ersten Bindungspartners einer Bindungsreaktion auf einer festen Oberfläche, wobei die feste Oberfläche aus paramagnetischen Teilchen besteht, wobei der erste Bindungspartner biotinyliert ist,
(b) Inkontaktbringen des ersten Bindungspartners mit der Lösung,
(c) Inkontaktbringen der Lösung mit einem zweiten Bindungspartner, wobei der zweite Bindungspartner an ein Calcium-sensitives lumineszentes Material konjugiert ist,
(d) nach einer Zeitdauer Mobilisieren der paramagnetischen Teilchen in einer vorbestimmten Richtung entlang einer Seite einer länglichen Matrix eines Einfangstreifens, um die Teilchen mit einem in Querrichtung auf dem Einfangstreifen angeordneten Streifen in Kontakt zu bringen, wobei auf dem Einfangstreifen Streptavidin auf dem Querstreifen immobilisiert ist, wobei der Querstreifen zusätzlich eine Calcium einschließende Verbindung enthält,
(e) Zulassen einer Zeitdauer, die ausreichend ist, damit die paramagnetischen Teilchen mit dem auf dem Querstreifen immobilisierten Streptavidin in Kontakt treten können,
(f) Aussetzen des Querstreifens des Einfangstreifens an einen Puls von ultraviolettem Licht, um die Freisetzung von Calcium aus der Calcium einschließenden Verbindung zu bewirken, und
(g) Messen der von dem Calcium-sensitiven lumineszenten Material emittierten Lumineszenz.

22. Verfahren nach Anspruch 21, wobei die Stufen (b) und (c) gleichzeitig ausgeführt werden.

23. Verfahren zum Durchführen eines Bindungstests, um das Vorliegen eines Analyten in einer Lösung zu detektieren, welches die folgenden Stufen umfaßt:
(a) Inkontaktbringen eines ersten Bindungspartners mit der Lösung, wobei der erste Bindungspartner biotinyliert ist,
(b) nach einer Zeitdauer Inkontaktbringen der Lösung mit einem zweiten Bindungspartner, wobei der zweite Bindungspartner an ein Calcium-sensitives lumineszentes Material konjugiert ist,
(c) nach einer weiteren Zeitdauer Mobilisieren der Bindungspartner in einer vorbestimmten Richtung entlang einer Seite einer länglichen Matrix eines Einfangstreifens, um die Bindungspartner mit einem in Querrichtung auf dem Einfangstreifen angeordneten Streifen in Kontakt zu bringen, wobei in dem Einfangstreifen Streptavidin auf dem Querstreifen immobilisiert ist, wobei der Querstreifen zusätzlich eine Calcium einschließende Verbindung enthält,
(d) Zulassen einer Zeitdauer, die ausreichend ist, damit die Bindungspartner mit dem auf dem Querstreifen immobilisierten Streptavidin in Kontakt treten können,
(e) Aussetzen des Querstreifens des Einfangstreifens an einen Puls von ultraviolettem Licht, um die Freisetzung von Calcium aus der Calcium einschließenden Verbindung zu bewirken, und
(f) Messen der von dem Calcium-sensitiven lumineszenten Material emittierten Lumineszenz.

24. Verfahren nach Anspruch 23, wobei die Stufen (a) und (b) gleichzeitig ausgeführt werden.

25. Verfahren nach einem der Ansprüche 21 bis 24, wobei der längliche Einfangstreifen einen quer verlaufenden Abschnitt aufweist, der mit Streptavidin und einer Calcium einschließenden Verbindung imprägniert ist.

26. Verfahren nach einem der Ansprüche 1 bis 25, wobei der Puls von ultraviolettem Licht und die Detektion der Chemilumineszenz in zeitaufgelöster Weise ausgeführt werden.

27. Verfahren nach einem der Ansprüche 1 bis 26, wobei die Lösung vor dem Puls von ultraviolettem Licht weniger als etwa 20 Nanomolar an Calcium enthält.

28. Länglicher Einfangstreifen für Bindungstests, wobei der Streifen einen quer verlaufenden Abschnitt aufweist, der mit Streptavidin und einer Calcium einschließenden Verbindung imprägniert ist.

29. Länglicher Einfangstreifen nach Anspruch 28, wobei der Einfangstreifen aus Nitrozellulose, Polyacrylamid, Polyamid oder irgendeinem anderen synthetischen oder natürlich vorkommenden Polymer gebildet ist.

30. Länglicher Einfangstreifen nach Anspruch 28 oder Anspruch 29, wobei der Einfangstreifen sich in einem Gehäuse befindet.

31. Länglicher Einfangstreifen nach Anspruch 30, wobei der Einfangstreifen in einem Träger als eine Testkartusche zur einmaligen Verwendung aufgenommen ist.

32. Länglicher Einfangstreifen nach einem der Ansprüche 28 bis 31, wobei die Calcium einschließende Verbindung aus der Gruppe ausgewählt ist, bestehend aus cis-1-(2-bis-(Carboxymethyl)-amino-5-(1-hydroxy-1-(2-nitro-4,5-methylendioxyphenyl)-methyl)-phenoxy)-2-(2-bis-(carboxymethyl)-amino-5-methylphenoxy)-cyclopentan, 1-[2-(Amino-5-(1-hydroxy-1-[2-nitro-4,5-methylendioxyphenyl]-methyl)-phenoxy]-2-)2'-amino-5'-methylphenoxy)-ethan-N,N,N',N'-tetraessigsäure, 1-(4,5-Dimethoxy-2-nitrophenyl)-1,2-diaminoethan-N,N,N',N'-tetraessigsäure und Nitrophenylethylen-bis-(oxyethylennitrilo)-tetraessigsäure.

33. Kunststoffkartusche zum Durchführen eines Bindungstests, um das Vorliegen eines Analyten in einer Lösung zu detektieren, welche folgendes umfaßt:
ein Gehäuse mit einer Aufnahme zum Aufnehmen einer Probe, ein Reservoir, welches einen biotinylierten ersten Bindungspartner, der auf paramagnetischen Teilchen immobilisiert ist, und einen zweiten Bindungspartner, der an ein Calcium-sensitives Chemilumineszenzmaterial konjugiert ist, enthält, einen länglichen Einfangstreifen innerhalb des Gehäuses und in Fluidverbindung mit dem Reservoir, wobei der Einfangstreifen einen quer verlaufenden Abschnitt aufweist, der mit einer Calcium einschließenden Verbindung und Streptavidin imprägniert ist, wobei der quer verlaufende Abschnitt durch eine Lichtbarriere geschützt ist.

34. Kunststoffbehälter nach Anspruch 33, wobei sich ein Filter zwischen der Aufnahme und dem Reservoir befindet.

35. Kunststoffkartusche nach Anspruch 33, wobei ein Filter vorgesehen ist, welcher ein Mittel zum Entfernen von Calcium enthält.

36. Kunststoffkartusche nach einem der Ansprüche 33 bis 35, wobei das Calcium-sensitive lumineszente Material Aequorin, Obeln, Mnemiopsin, Berovin, Pholasin, Luciferasen oder Photoproteine, isoliert aus Pelagia, Cypridina und Ostracoda, ist.

37. Vorrichtung zum Durchführen eines Bindungstests, umfassend ein Gehäuse, in welchem folgendes aufgenommen ist: (a) eine Aufnahme, um die Kunststoffkartusche nach einem der Ansprüche 33 bis 36 aufzunehmen, (b) ein Mittel zum Entfernen der Lichtschutzschicht über dem Querstreifen, (c) ein Elektromagnet, um ein magnetisches Feld bereitzustellen, (e) eine Ultraviolettlichtquelle, um Licht auf einen zuvor ausgewählten Abschnitt des Einfangstreifens zu projizieren, und (f) ein Photomultiplier, der so angeordnet ist, daß er von dem zuvor ausgewählten Abschnitt des Einfangstreifens emittiertes Licht aufnimmt.

38. Vorrichtung nach Anspruch 37, wobei der Elektromagnet mehrere magnetische Felder entlang der Kunststoffkartusche erzeugt.

39. Vorrichtung nach Anspruch 37 oder Anspruch 38, wobei die Ultraviolettlichtquelle Licht im Bereich von 250-400 nm liefert.

40. Vorrichtung nach einem der Ansprüche 37 bis 39, wobei der Photomultiplier Licht im Bereich von 400-600 nm detektiert.

## Revendications

1. Méthode pour effectuer une analyse de liaison afin de détecter la présence d'un analyte dans une solution, comprenant les étapes consistant à :
(a) mettre en contact un premier partenaire de liaison avec ladite solution, ledit premier partenaire de liaison étant conjugué à une substance chimioluminescente sensible au calcium ;
(b) après une période de temps, mobiliser le premier partenaire de liaison dans une direction prédéterminée le long d'une face d'une matrice allongée d'une bandelette de capture de manière à mettre en contact le premier partenaire de liaison avec une bande située transversalement sur ladite bandelette de capture, ladite bande transversale comprenant un second partenaire de liaison immobilisé et contenant un composé emprisonnant le calcium ;
(c) laisser s'écouler une période de temps suffisante pour que le premier partenaire de liaison entre en contact avec ledit second partenaire de liaison immobilisé sur ladite bande transversale ;
(d) exposer ladite bande transversale de ladite bandelette de capture à une impulsion de lumière ultraviolette pour provoquer la libération du calcium du composé de calcium emprisonné ; et
(e) mesurer la luminescence émise par la substance luminescente sensible au calcium.

2. Méthode suivant la revendication 1, pour effectuer une analyse de liaison afin de détecter la présence d'un analyte dans une solution, comprenant les étapes consistant à :
(a) mettre en contact ladite solution avec un premier partenaire de liaison d'une réaction de liaison, ledit premier partenaire de liaison étant immobilisé sur une surface solide, ladite surface solide consistant en particules paramagnétiques et ledit premier partenaire de liaison étant conjugué à une substance luminescente sensible au calcium ;
(b) après une période de temps, mobiliser les particules paramagnétiques dans une direction prédéterminée le long d'une face d'une matrice allongée d'une bandelette de capture de manière à mettre en contact les particules avec une bande d'un second partenaire de liaison située transversalement sur ladite bandelette de capture, ladite bandelette de capture comprenant un second partenaire de liaison immobilisé sur ladite bande transversale, ladite bande transversale contenant en outre un composé emprisonnant le calcium,
(c) laisser s'écouler une période de temps suffisante pour que les particules paramagnétiques entrent en contact avec ledit second partenaire de liaison immobilisé sur ladite bande transversale,
(d) exposer ladite bande transversale de ladite bandelette de capture à une impulsion de lumière ultraviolette pour provoquer la libération du calcium du composé emprisonnant le calcium ; et
(e) mesurer la luminescence émise par la substance luminescente sensible au calcium.

3. Méthode suivant la revendication 2, ladite méthode étant une analyse immunologique pour détecter et quantifier un antigène, une analyse immunologique pour détecter et quantifier un anticorps, ou une analyse d'hybridation d'acide nucléique pour la détection et la quantification d'une séquence particulière d'un acide nucléique.

4. Méthode suivant l'une quelconque des revendications 1 à 3, dans laquelle la solution est prétraitée avant la mise en contact de la substance luminescente sensible au calcium dans l'étape (a).

5. Méthode suivant la revendication 4, dans laquelle la solution est filtrée pour éliminer le calcium, le filtre contenant un agent pour l'élimination du calcium.

6. Méthode suivant l'une quelconque des revendications 1 à 5, dans laquelle la solution consiste en du sang entier, ledit sang entier étant prétraité par filtration avant mise en contact avec la substance luminescente sensible au calcium.

7. Méthode suivant l'une quelconque des revendications 1 à 6, dans laquelle la substance luminescente sensible au calcium est l'aéquorine, l'obéine, la mnémiopsine, la bérovine, la pholasine, des luciférases ou des photoprotéines isolées de Pelagia, de Cypridina et d'ostracodes.

8. Méthode suivant l'une quelconque des revendications 1 à 7, dans laquelle la lumière ultraviolette est sous forme d'une impulsion de lumière dans la plage de 250 à 400 nm, et la luminescence est mesurée par un photomultiplicateur.

9. Méthode suivant la revendication 8, dans laquelle la substance luminescente sensible au calcium est l'aéquorine, et dans laquelle le photomultiplicateur détecte la lumière de 400 à 600 nm et est protégé contre le champ magnétique.

10. Méthode suivant l'une quelconque des revendications 1 à 9, dans laquelle la bandelette allongée de capture est formée de nitrocellulose, de polyacrylamide ou de n'importe quel autre polymère naturel ou synthétique.

11. Méthode suivant la revendication 10, dans laquelle la bandelette allongée de capture comporte une bande transversale avec le second partenaire de liaison immobilisé et est imprégnée avec un composé emprisonnant le calcium.

12. Méthode suivant l'une quelconque des revendications 1 à 11, dans laquelle le composé emprisonnant le calcium est chargé de calcium en excès de la quantité stoechiométrique pour ladite substance luminescente sensible au calcium.

13. Méthode suivant l'une quelconque des revendications 1 à 12, dans laquelle le composé emprisonnant le calcium est choisi dans le groupe consistant en le cis-1-(2-bis(carboxyméthyl)amino-5-(1-hydroxy-1-(2-nitro-4,5-méthylènedioxyphényl)méthyl)-phénoxy)-2-(2-bis(carboxyméthyl)amino-5-méthylphénoxy)-cyclopentane, l'acide 1-[2-amino-5-(1-hydroxy-1-[2-nitro-4,5-méthylènedioxyphényl]méthyl)-phénoxy]-2-)2'-amino-5'-méthylphénoxy)éthane-N,N,N',N'-tétraacétique, l'acide 1-(4,5-diméthoxy-2-nitrophényl)-1,2-diaminoéthane-N,N,N',N'-tétraacétique et l'acide nitrophényl-éthylènebis-(oxyéthylènenitrilo)tétraacétique.

14. Méthode suivant l'une quelconque des revendications 1 à 13, qui est une analyse immunologique pour la détection et la quantification d'un antigène.

15. Méthode suivant l'une quelconque des revendications 1 à 13, qui est une analyse immunologique pour la détection et la quantification d'un anticorps.

16. Méthode suivant l'une quelconque des revendications 1 à 13, dans laquelle l'analyse de liaison est une analyse d'hybridation d'acide nucléique pour la détection et la quantification d'une séquence particulière d'un acide nucléique.

17. Méthode suivant l'une quelconque des revendications 1 à 16, dans laquelle la substance luminescente sensible au calcium est l'aéquorine.

18. Méthode suivant l'une quelconque des revendications 1 à 17, dans laquelle la source de lumière ultraviolette émet une impulsion de lumière dans la plage de 250 à 400 nm.

19. Méthode suivant l'une quelconque des revendications 1 à 18, dans laquelle la luminescence est mesurée par un photomultiplicateur.

20. Méthode suivant l'une quelconque des revendications 1 à 19, dans laquelle la substance luminescente sensible au calcium est l'aéquorine et le photomultiplicateur détecte la lumière de 400 à 600 nm et est protégé contre le champ magnétique.

21. Méthode pour effectuer une analyse de liaison afin de détecter la présence d'un analyte dans une solution, comprenant les étapes consistant à :
(a) immobiliser un premier partenaire de liaison d'une réaction de liaison sur une surface solide, ladite surface solide consistant en particules paramagnétiques, ledit premier partenaire de liaison étant biotinylé ;
(b) mettre en contact ledit premier partenaire de liaison avec ladite solution ;
(c) mettre en contact la solution avec un second partenaire de liaison, ledit second partenaire de liaison étant conjugué à une substance luminescente sensible au calcium ;
(d) après une période de temps, mobiliser les particules paramagnétiques dans une direction prédéterminée le long d'une face d'une matrice allongée d'une bandelette de capture de manière à mettre en contact les particules avec une bande située transversalement sur ladite bandelette de capture, ladite bandelette de capture comprenant de la streptavidine immobilisée sur ladite bande transversale, ladite bande transversale contenant en outre un composé emprisonnant le calcium,
(e) laisser s'écouler une période de temps suffisante pour que les particules paramagnétiques entrent en contact avec ladite streptavidine immobilisée sur ladite bande transversale,
(f) exposer ladite bande transversale de ladite bandelette de capture à une impulsion de lumière ultraviolette pour effectuer la libération du calcium du composé emprisonnant le calcium ; et
(g) mesurer la luminescence émise par la substance luminescente sensible au calcium.

22. Méthode suivant la revendication 21, dans laquelle les étapes (b) et (c) sont mises en oeuvre simultanément.

23. Méthode pour effectuer une analyse de liaison afin de détecter la présence d'un analyte dans une solution, comprenant les étapes consistant à :
(a) mettre en contact un premier partenaire de liaison avec ladite solution, ledit premier partenaire de liaison étant biotinylé ;
(b) après une période de temps, mettre en contact la solution avec un second partenaire de liaison, ledit second partenaire de liaison étant conjugué à une substance luminescente sensible au calcium ;
(c) après une période de temps supplémentaire, mobiliser les partenaires de liaison dans une direction prédéterminée le long d'une face d'une matrice allongée d'une bandelette de capture de manière à mettre en contact les partenaires de liaison avec une bande située transversalement sur ladite bandelette de capture, ladite bandelette de capture comprenant de la streptavidine immobilisée sur ladite bande transversale, ladite bande transversale contenant en outre un composé emprisonnant le calcium,
(d) laisser s'écouler une période de temps suffisante pour que les partenaires de liaison entrent en contact avec ladite streptavidine immobilisée sur ladite bande transversale,
(e) exposer ladite bande transversale de ladite bandelette de capture à une impulsion de lumière ultraviolette pour provoquer la libération du calcium du composé emprisonnant le calcium ; et
(f) mesurer la luminescence émise par la substance luminescente sensible au calcium.

24. Méthode suivant la revendication 23, dans laquelle les étapes (a) et (b) sont mises en oeuvre simultanément.

25. Méthode suivant l'une quelconque des revendications 21 à 24, dans laquelle la bandelette allongée de capture a une section transversale imprégnée de streptavidine et d'un composé emprisonnant le calcium.

26. Méthode suivant l'une quelconque des revendications 1 à 25, dans laquelle l'impulsion de lumière ultraviolette et la détection de chimioluminescence sont effectuées de manière séparée dans le temps.

27. Méthode suivant l'une quelconque des revendications 1 à 26, dans laquelle la solution contient une quantité inférieure à 20 nanomolaire de calcium avant l'impulsion de lumière ultraviolette.

28. Bandelette allongée de capture pour des analyses de liaison, ladite bandelette ayant une section transversale imprégnée de streptavidine et d'un composé emprisonnant le calcium.

29. Bandelette allongée de capture suivant la revendication 28, ladite bandelette de capture étant formée de nitrocellulose, de polyacrylamide, de polyamide ou de n'importe quel autre polymère synthétique ou naturel.

30. Bandelette allongée de capture suivant la revendication 28 ou la revendication 29, ladite bandelette de capture étant présente dans un boîtier.

31. Bandelette allongée de capture suivant la revendication 30, ladite bandelette de capture étant logée dans un support sous forme d'une cartouche de test à usage unique.

32. Bandelette allongée de capture suivant l'une quelconque des revendications 28 à 31, dans laquelle le composé emprisonnant le calcium est choisi dans le groupe consistant en le cis-1-(2-bis(carboxyméthyl)amino-5-(1-hydroxy-1-(2-nitro-4,5-méthylènedioxyphényl)méthyl)phénoxy)-2-(2-bis-(carboxyméthyl)amino-5-méthylphénoxy)cyclopentane, l'acide 1-[2-amino-5-(1-hydroxy-1-[2-nitro-4,5-méthylènedioxyphényl]méthyl)-phénoxy]-2-)2'-amino-5'-méthylphénoxy)éthane-N,N,N',N'-tétraacétique, l'acide 1-(4,5-diméthoxy-2-nitrophényl)-1,2-diaminoéthane-N,N,N',N'-tétraacétique et l'acide nitrophényl-éthylènebis(oxyéthylènenitrilo)tétraacétique.

33. Cartouche en matière plastique pour effectuer une analyse de liaison afin de détecter la présence d'un analyte dans une solution, comprenant :
un boîtier avec un réceptacle pour recevoir un échantillon, un réservoir contenant un premier partenaire de liaison biotinylé immobilisé sur des particules paramagnétiques et un second partenaire de liaison conjugué à une substance chimioluminescente sensible au calcium, une bandelette allongée de capture dans le boîtier et en communication par fluide avec le réservoir, ladite bandelette de capture ayant une section transversale imprégnée d'un composé emprisonnant le calcium et de streptavidine, ladite section transversale étant protégée avec un écran faisant barrage à la lumière.

34. Cartouche en matière plastique suivant la revendication 33, dans laquelle il existe un filtre entre le réceptacle et le réservoir.

35. Cartouche en matière plastique suivant la revendication 33, dans laquelle il existe un filtre contenant un agent pour l'élimination du calcium.

36. Cartouche en matière plastique suivant l'une quelconque des revendications 33 à 35, dans laquelle la substance luminescente sensible au calcium est l'aéquorine, l'obéine, la mnémiopsine, la bérovine, la pholasine, des luciférases ou des photoprotéines isolées de Pelagia, de Cypridina et d'ostracodes.

37. Appareil pour la mise en oeuvre d'une analyse de liaison, comprenant un boîtier entourant (a) un réceptacle pour recevoir la cartouche en matière plastique de l'une quelconque des revendications 33 à 36 ; (b) un moyen pour éliminer la couche de protection contre la lumière sur la bande transversale ; (c) un électroaimant pour fournir un champ magnétique ; (e) une source de lumière ultraviolette pour projeter de la lumière sur une partie choisie préalablement de la bandelette de capture ; et (f) un photomultiplicateur disposé pour recevoir la lumière émise par la partie choisie préalablement de la bandelette de capture.

38. Appareil suivant la revendication 37, dans lequel l'électroaimant projette des champs magnétiques multiples le long de la cartouche en matière plastique.

39. Appareil suivant la revendication 37 ou la revendication 38, dans lequel la source de lumière ultraviolette fournit de la lumière dans la plage de 250 à 400 nm.

40. Appareil suivant l'une quelconque des revendications 37 à 39, dans lequel le photomultiplicateur détecte la lumière dans la plage de 400 à 600 nm.
